# EUROPÄISCHE PATENTSCHRIFT

(11) **EP 0 912 188 B1**
(45) Veröffentlichungstag und Bekanntmachung des Hinweises auf die Patenterteilung: **29.01.2003**
(21) Anmeldenummer: 97931723.7
(22) Anmeldetag: 09.07.1997
(51) Int. Cl.: A61K 35/14, A61P 31/18, A61M 1/38, B01D 15/08, C12N 7/02

(54) **SELEKTIVES ADSORBENS ZUR BINDUNG VON RETROVIREN**
SELECTIVE ADSORBENT FOR BINDING RETROVIRUSES
ADSORBANT SELECTIF DESTINE A FIXER DES RETROVIRUS

(30) Priorität: 12.07.1996 DE 19628190; 23.06.1997 DE 19727830
(43) Veröffentlichungstag der Anmeldung: 06.05.1999
(73) Patentinhaber: IMTEC IMMUNDIAGNOSTIKA GMBH, 16341 Zepernick (DE); Fresenius AG, 61350 Bad Homburg (DE)
(72) Erfinder: HIEPE, Falk, D-12557 Berlin (DE); SCHÖSSLER, Werner, D-16341 Schwanebeck (DE); PFÜLLER, Barbara, D-16341 Zepernick (DE); LEINENBACH, Hans, Peter, D-66636 Tholey (DE); OTTO, Veit, D-66606 St. Wendel (DE); HEPPER, Martin, D-67435 Neustadt (DE); MONTAG-LESSING, Thomas, D-64283 Darmstadt (DE)
(74) Vertreter: Baumbach, Friedrich, Dr.
(86) Internationale Anmeldenummer: DE9701446
(87) Internationale Veröffentlichungsnummer: WO98002174

(56) Entgegenhaltungen:
- EP-A- 0 222 146
- WO-A-96/16666
- WO-A-96/21675
- Z. PROH SZKA ET AL.: "INTERACTION OF COMPLEMENT AND SPECIFIC ANTIBODIES WITH THE EXTERNAL GLYCOPROTEIN 120 OF HIV-1." IMMUNOLOGY., Bd. 85, Nr. 2, Juni 1995, OXFORD, GB, Seiten 184-189, XP002044878

## Beschreibung

Die Erfindung betrifft die Verwendung eines selektiven Adsorbens zur Herstellung eines Arzneimittels zur Bindung von Retroviren, insbesondere von HI-Viren. Anwendungsgebiete der Erfindung sind die Medizin und die pharmazeutische Industrie.

Für den Verlauf und die Prognose der HIV-Erkrankung spielt die Viruslast (virus load) eine entscheidende Rolle. Eine deutliche Verringerung der Anzahl der Viruspartikel insbesondere in der ersten Phase der Erkrankung bzw. im Endstadium wäre sinnvoll und wünschenswert.
Durch die heute bekannten medikamentösen Kombinationstherapien können effektive Virusabsenkungen bis z.T. unterhalb der Nachweisgrenze erreicht werden. Sie setzen aber eine sehr hohe Compliance des Patienten, vor allem eine hohe Einnahmedisziplin voraus. Es gibt demgegenüber Angaben, daß 30-45 % der Betroffenen die Medikamente nicht regelmäßig einnehmen. Daraus erwächst in verstärktem Maß die Gefahr der Entwicklung von Resistenzen. Ein weiteres Problem besteht darin, daß die Betroffenen bereits zu einem Zeitpunkt große Mengen von Medikamenten mit zahlreichen, nicht unerheblichen Nebenwirkungen einnehmen müssen, da sie noch bei subjektivem Wohlbefinden sind. Zu den Nebenwirkungen gehören u.a. Knochenmarksdepressionen, periphere Neuropathien und Pankreatitiden.

Die Bildung zirkulierender Immunkomplexe (Antigen/Antikörper-Komplexe) stellt einen physiologischen Abwehrmechanismus zur Eliminierung endogener oder exogener Antigene dar. Hierzu werden die zirkulierenden Immunkomplexe an die Subkomponente C1_{q} der ersten Komplementkomponente gebunden und führen im folgenden zur Aktivierung des Komplementsystems und zur Auslösung der Komplementkaskade, wobei durch Freisetzung einer Vielzahl von biologisch aktiven Substanzen der Entzündungsprozeß initiiert wird.

Unter Nutzung dieser biospezifischen Wechselwirkung gelang es, zirkulierende Immunkomplexe aus Körperflüssigkeiten mit einem an einen Träger gebundenen Protein C1_{q} selektiv und spezifisch zu binden. Damit können insbesondere Autoimmunerkrankungen, bei denen Immunkomplexe eine pathogenetische Rolle spielen, wirkungsvoll therapiert werden (DD 241 791).

Es ist ferner bekannt, daß Glykoproteine des HI-Virus, wie gp 41 oder gp 120, mit dem Protein C1_{q} in Wechselwirkung treten können(Stoiber et. al., Molecular Immunology, Vol. 32(1995), S. 371-374).

Diese Glykoproteine sind in der Membran des HI-Virus eingebettet und stehen im physiologischen Umfeld primär nicht zur Verfügung, d. h. eine Detektion der Viren läßt sich durch eine Reaktion mit diesen Glykoproteinen nicht erreichen. Verantwortlich hierfür scheinen sterische Hinderungen und/oder geringe Affinitäten zu sein. C1_{q} ist eine Subkomponente der 1. Komplementkomponente, die sich aus den Proteinen C1_{q,} C1ᵣ und C1ₛ zusammensetzt. Damit ist die Bindung von HI- und anderen Viren schon aus sterischen Gründen an das Cl-Komplex vorliegende C1_{q} nicht möglich.

Eine Bindung des in Körperflüssigkeiten in außerordentlich geringer Konzentration [Zahlenangabe liegt vor] vorliegenden freien C1_{q} an HI- oder andere Viren wurde bisher bei entsprechenden AIDS-Patienten nicht nachgewiesen. Es ist lediglich davon auszugehen, daß ein Teil der HI-Viren als Immunkomplex vorliegt und dieser sich in bekannter Art und Weise an das C1_{q} zu binden vermag. Diese Bindung entspricht dem bekannten Stand der Technik, die auch zur Entfernung zirkulierender Immunkomplexe mittels C1_{q} Adsorber genutzt wurde (DD 241 791). Hingegen ist eine direkte Bindung von HI-Viren an in flüssiger Phase und in physiologischer Umgebung befindlichem C1_{q} nicht bekannt.

Sie liegen gewöhnlicherweise nicht als Immunkomplex vor, so daß ä priori auch keine Bindung an C1_{q} zu erwarten ist.

Ziel der Erfindung ist die Bekämpfung von retroviral bedingten Erkrankungen, insbesondere von AIDS. Die Aufgabe besteht darin, Retroviren, insbesondere HI-Viren durch extrakorporale Immunadsorption aus Körperflüssigkeiten zu entfernen und weiterhin Retroviren insbesondere HI-Viren in vitro und/oder in vivo an eine feste Phase (Träger) zu binden, wobei die feste Phase sterilisierbar, biokompatibel, mikrobiell schwer angreifbar und stabil sein soll.

Es wurde gefunden, daß sich HI-Viren in einer hohen Affinität und Selektivität an immobilisiertes C1_{q} binden, wobei die festen Träger eine Partikelgröße von 10 -1000 µm und eine Porosität > 10 nm aufweisen.
Auf der Grundlage dieses Befundes wird die Aufgabe der Erfindung gemäß den Ansprüchen 1 bis 10 realisiert.

Erfindungsgemäß wird C1_{q} - als natürlicher biologischer Ligand - an ein aminiertes Polyhydroxymethacrylat über Glutaraldehyd kovalent gekoppelt und dieses trägergebundene C1_{q} wird zur Bindung und Entfernung von Retroviren, insbesondere HI-Viren und Retrovirus-haltigen, insbesondere HI-Virus-haltigen Immunkomplexen aus Körperflüssigkeiten eingesetzt.

Die Verwendung des erfindungsgemäßen Adsorbens erlaubt bei ein- bis mehrmaliger Anwendung eine nahezu vollständige Entfernung von Retroviren aus Körperflüssigkeiten und trägt damit wesentlich zur erfolgreichen Behandlung von retroviralen Erkrankungen wie AIDS bei.

Die Behandlung mit einem, auf oben beschriebenen Prinzip beruhenden extrakorporalen Verfahren ist nahezu nebenwirkungsfrei und eine sinnvolle Ergänzung bzw. Alternative zur medikamentösen Therapie.

### Beispiel 1:

Die C1_{q}-beschichtete Matrix (2 ml) wird in eine Chromatographiesäule (Durchmesser:20 mm) gefüllt und mit 20 ml RPMI 1640-Medium, 20 mM EDTA enthaltend, gespült. Anschließend werden 1ml Zellkulturüberstände der T-Zelllinie HIV 1/LAI mit 10⁷ - 10⁸ RNA-Kopien/ml auf die Säule aufgetragen und 10 min inkubiert. Danach wird mit 3 ml und 10 ml des oben genannten Puffer eluiert und in den Fraktionen die Menge der Viruspartikel durch Auffangen der Eluate und Messung der RNA-Kopien/ml mit dem AMPLICOR HIV Monitor™-Assay bestimmt. Die Bindungsrate wird durch den Vergleich mit der aufgetragenen Probe ermittelt.

### Beispiel 2:

Wie im Beispiel 1 beschrieben, wird eine 2 ml C1_{q}beschichtete Matrix enthaltende Chromatographiesäule mit 20 ml RPMI 1640-Medium, 20 mM EDTA enthaltend, gespült. Anschließend werden 1ml Zellkulturüberstände der T-Zelllinie HIV 1/LAI mit 10⁴ - 10⁵ RNA-Kopien/ml auf die Säule aufgetragen und 10 min inkubiert. Danach wird wie im Beispiel 1 weiter verfahren.

### Beispiel 3:

Wie im Beispiel 1 und 2 beschrieben, wird eine 2 ml C1_{q}beschichtete Matrix enthaltende Chromatographiesäule mit 20 ml RPMI 1640-Medium gespült. Auf diese Säule wird 1ml Serum bzw. Heparinplasma eines HIV-infizierten Patienten mit 12 x 10³ RNA-Kopien/ml bzw. 15 x 10³ RNA-Kopien/ml aufgetragen und 10 min inkubiert. Anschließend wird zunächst mit 3 ml und anschließend mit 10 ml RPMI 1640-Medium eluiert und jeweils aus einem Aliquot die Menge der Viruspartikel durch Messung der RNA-Kopien/ml mit dem AMPLICOR HIV Monitor™-Assay bestimmt. Die Bindungsrate wird durch den Vergleich mit der auf getragenen Probe ermittelt.

In Tabelle 1 sind die Ergebnisse der Ausführungsbeispiele 1-3 zusammengefaßt:

**Tabelle 1:**

| Probe | vor Adsorption | nach Adsorption | Bindungsrate % |
|---|---|---|---|
| Zellkulturüberstand 10⁷ - 10⁸ HIV-RNA-Kopien/ml | 10⁷ | 3,6 x 10⁶ | 64 |
| Zellkulturüberstand 10⁴ - 10⁵ HIV-RNA-Kopien/ml | 135 000 | 27 000 | 80 |
| Serum (HIV-Patient) 12 x 10³ RNA-Kopien/ml | 12062 | 3681 | 69,5 |
| Heparinplasma (HIV-Patient) 15 x 10³ RNA-Kopien/ml | 15741 | 6270 | 60,2 |

### Beispiel 4:

Die C1_{q}-beschichtete Matrix (2 ml) wird in eine Chromatographiesäule (Durchmesser: 20 mm) gefüllt und mit 20 ml D.A.L.I.-Primerlösung (ACDA im Verhältnis 1:20 enthaltend) gespült. Anschließend werden 0,5 ml EDTA-Plasma eines HIV-infizierten Patienten mit 40 x 10³ RNA-Kopien/ml aufgetragen und 10 min inkubiert. Danach wird mit 3 ml und 10 ml isotoner NaCl-Lösung eluiert und in den Fraktionen die Menge der Viruspartikel durch Auffangen der Eluate und Messung der RNA-Kopien/ml mit dem AMPLICOR IIIV Monitor™-Assay bestimmt. Die Bindungsrate wird durch den Vergleich mit der auf getragenen Probe ermittelt.

Der gleiche Versuch wird in identischer Weise durchgeführt mit dem Unterschied, daß das Patientenplasma mit C1_{q} in einer Konzentration vorinkubiert wird, die der Beschichtung der festen Phase entspricht.

| Probe | Bindungsrate (%) |
|---|---|
| EDTA-Plasma | 96,8 |
| EDTA-Plasma plus C1q | 93,6 |

Die gemessene Differenz liegt dabei innerhalb der Schwankungsbreite der PCR-Methode.

Im Ergebnis der beschriebenen Versuche ist davon auszugehen, daß freies Virus bzw. virushaltige Immunkomplexe besser an festphasengebundenes C1_{q} als an frei im Serum befindliches C1_{q} binden. Ursache dafür ist die durch die Kopplung an die feste Phase vermutlich erreichte bessere Zugänglichkeit der CLR-Region des C1_{q}, die die Bindungsregion für das Virus darstellt.

### Beispiel 5:

Die C1q-beschichtete Matrix (2 ml) wird in eine Chromatographiesäule (Durchmesser: 20mm) gefüllt und mit 20 ml D.A.L.I.-Primerlösung (ACDA im Verhältnis 1:20 enthaltend) gespült. Anschließend werden 0,5 ml EDTA-Plasma eines HIV-infizierten Patienten mit 347 x 10³ bzw. 1,292 x 10⁶ RNA-Kopien/ml aufgetragen und 10 min inkubiert. Danach wird mit 3 ml und 10 ml isotoner NaCl-Lösung eluiert und in den Fraktionen die Menge der Viruspartikel durch Auffangen der Eluate und Messung der RNA-Kopien/ml mit dem AMPLICOR HIV Monitor™- Assay bestimmt. Die Bindungsrate wird durch den Vergleich mit der aufgetragenen Probe ermittelt. Weiterhin wurde eine dritte Fraktion von 2 ml Volumen aufgefangen, der ebenfalls ein Aliquot zur Bestimmung der Viruszahl entnommen wurde. Mit der ersten aufgefangenen Fraktion, die erfahrungsgemäß noch Viren in den oben angegebenen Mengen enthielt, wurde in diesem Experiment eine Rechromografie durchgeführt.

| RNA-Kopien/ml | Bindungsrate [%] | Bindungsrate [%] nach Rechromatografie |
|---|---|---|
| 347 x 10³ | 66 | 94 |
| 1,292 x 10⁶ | 84 | 77,5 |

## Patentansprüche

1. Verwendung eines selektiven Adsorbens zur Herstellung eines Arzneimittels zur Bindung von Retroviren, insbesondere HI-Viren, **dadurch gekennzeichnet, daß** das Protein C1_{q} an feste Träger gebunden wird, deren Partikelgröße 10 - 1000 µm beträgt und deren Porosität > 10 nm ist, und daß nach Bindung der Retroviren an das Protein C1_{q} der festen Phase durch Behandlung mit der Körperflüssigkeit, insbesondere Blut, Plasma, Serum, und Abtrennung der festen Phase, eine Regenerierung der festen Phase durch Behandlung mit nicht denaturierenden Medien und Wiederverwendung derselben erfolgt.

2. Verwendung nach Anspruch 1, dadurch gekenn-zeichnet, daß das für die Immobilisierung eingesetzte C1_{q} humanen oder tierischen Ursprungs ist.

3. Verwendung nach Anspruch 1 oder 2, **dadurch gekennzeichnet, daß** das für die Immobilisierung eingesetzte C1_{q} rekombinanten Ursprungs ist.

4. Verwendung nach Anspruch 1 bis 3, **dadurch gekennzeichnet, daß** die nicht denaturierenden Medien gepufferte Lösungen hoher Ionenstärke, chaotropische Reagenzien oder aber stark saure bzw. alkalische Lösungen sind.

5. Verwendung nach Anspruch 1 bis 4, **dadurch gekennzeichnet, daß** die eingesetzen festen Phasen aus sphärischen, flächenförmigen oder faserförmigen Formkörpern bestehen oder aber als dünne Schichten, Filme oder Lacke auf anderen Trägern aufgebracht sind.

6. Verwendung nach Anspruch 1 bis 5, **dadurch gekennzeichnet, daß** die Formkörper durch Packung, Verweben, Sinterung oder durch Bindemittel zu vorzugsweise flächenförmigen Trägermaterialien oder Kolonnenpackungen zusammengestellt sind.

7. Verwendung nach Anspruch 1 bis 6, **dadurch gekennzeichnet, daß** als feste Träger organische Polymere, anorganische Polymere oder natürliche Polymere verwendet werden.

8. Verwendung nach Anspruch 7, **dadurch gekennzeichnet, daß** als organische Polymere sphärische Polyhydroxyethylmethacrylate zum Einsatz gelangen.,

9. Verwendung nach Anspruch 7, **dadurch gekennzeichnet, daß** die anorganischen Polymere siliziumdioxidhaltige Trägermaterialien sind.

10. Verwendung nach Anspruch 1 bis 5 und/oder 6 und 7-9, **dadurch gekennzeichnet, daß** als Brückenverbindungen homooder heterobifunktionelle Reagenzien und andere Verbindungen, wie Epichlorhydrin, CNBr oder Carbodiimide mit und ohne Spacer eingesetzt werden.

## Claims

1. Use of a selective adsorbent for the production of a medication for binding retroviruses, in particular HI viruses, wherein the protein Cl_{q} is bound to solid carriers, the particle size of which amounts to 10 - 1000 µm and the porosity of which is > 10 nm, and wherein, after binding of the retroviruses to the protein Cl_{q} of the solid phase by treatment with the body fluid, in particular blood, plasma, serum, and separation of the solid phase, a regeneration of the solid phase takes place by treatment with non-denaturing media and re-use of the same.

2. Use according to Claim 1, wherein the Cl_{q} used for the immobilisation is of human or animal origin.

3. Use according to Claim 1 or 2, wherein the Cl_{q} used for the immobilisation is of recombinant origin.

4. Use according to Claims 1 to 3, wherein the non-denaturing media are buffered solutions of high ion strength, chaotropic reagents or highly acid or alkaline solutions.

5. Use according to Claims 1 to 4, wherein the solid phases used comprise spherical, planiform or fibre-shaped bodies or are attached to other carriers as thin layers, films or lacquers.

6. Use according to Claims 1 to 5, wherein the shaped bodies have been put together by packaging, weaving, sintering or by binding agents to form preferably planiform carrier materials or packages of columns.

7. Use according to Claims 1 to 6, wherein organic polymers, inorganic polymers or natural polymers are used as solid carriers.

8. Use according to Claim 7, wherein spherical polyhydroxyethylmethacrylates are used as organic polymers.

9. Use according to Claim 7, wherein the inorganic polymers are carrier materials containing silicium oxide.

10. Use according to Claims 1 to 5 and/or 6 and 7 to 9, wherein homo- or hetero-bifunctional reagents and other compounds such as epichlorhydrine, CNBr or carbodiimides with and without spacers are used as bridge connections.

## Revendications

1. Emploi d'un adsorbant sélectif pour la fabrication d'un médicament dans le but de lier des rétrovirus, en particulier des virus I.H.V., se caractérisant par le fait que la protéine Cl_{q} sera liée à des porteurs fixes dont la taille de particules s'élève entre 10 et 1000 µm et dont la porosité est > 10 nm et par le fait que après la liaison des rétrovirus à la protéine Cl_{q} de la phase solide par traitement avec des liquides corporels, en particulier du sang, du plasma, du sérum et par séparation de la phase solide, une régénération de la phase solide se fera par le traitement avec des milieux non dénaturés et par réemploi de ces derniers.

2. Emploi selon la revendication 1, se caractérisant par le fait que la protéine Cl_{q} utilisée pour l'immobilisation est d'origine humaine ou animale.

3. Emploi selon la revendication 1 ou 2, se caractérisant par le fait que la protéine Cl_{q} utilisées pour l'immobilisation est d'origine recombinante.

4. Emploi selon la revendication 1 à 3, se caractérisant par le fait que les milieux non dénaturés sont des solutions tamponnées de force ionique élevée, des réactifs chaotropiques ou bien des solutions fortement acides resp. alcalines.

5. Emploi selon la revendication 1 à 4, se caractérisant par le fait que les phases solides employées se composent de corps façonnés sphériques, planiformes ou fibreux ou bien sont appliquées sous forme de couches fines, films ou laques sur d'autres porteurs.

6. Emploi selon la revendication 1 à 5, se caractérisant par le fait que les corps façonnés sont assemblés par tassement, entrelacement, frittage ou par l'intermédiaire de liants pour former des matériaux porteurs de préférence planiformes ou des garnissages de colonne.

7. Emploi selon la revendication 1 à 6, se caractérisant par le fait que des polymères organiques, des polymères anorganiques ou des polymères naturels sont utilisés en tant que porteurs solides.

8. Emploi selon la revendication 7, se caractérisant par le fait que des poly(hydroxyéthyl)methacrylates sphériques sont utilisés en tant que polymères organiques.

9. Emploi selon la revendication 7, se caractérisant par le fait que les polymères anorganiques sont des matériaux porteurs contenant des dioxydes de silicium.

10. Emploi selon la revendication 1 à 5 et/ou 6 et 7-9, se caractérisant par le fait que des réactifs homobi- ou hétérobifonctionnels sont utilisés en tant que liaisons pontées et d'autres liaisons, telles que l'épichlorhydrine, le CNBr ou le carbodiimide sont utilisées avec et sans espaceur.
